# EUROPEAN PATENT SPECIFICATION

(11) **EP 2 142 036 B1**
(45) Date of publication and mention of the grant of the patent: **29.06.2011**
(21) Application number: 08804198.3
(22) Date of filing: 15.09.2008
(51) Int. Cl.: A46B 3/18, A46B 11/00, A46B 9/06

(54) **A MEDICAL CLEANING TOOL**
MEDIZINISCHES REINIGUNGSWERKZEUG
OUTIL DE NETTOYAGE MÉDICAL

(30) Priority: 27.12.2007 SE 0702890; 27.12.2007 US 9195 P; 16.06.2008 US 129275 P
(43) Date of publication of application: 13.01.2010
(73) Proprietor: Corticalis AS, 1450 Nesoddtangen (NO)
(72) Inventor: LYNGSTADAAS, Staale Petter, 1450 Nesoddtangen (NO); ELLINGSEN, Jan Eirik, 1356 Bekkestua (NO); WOHLFAHRT, Johan Caspar, 0287 Oslo (NO)
(74) Representative: VALEA AB
(86) International application number: PCT/EP2008/062237
(87) International publication number: WO 2009/083281

(56) References cited:
- EP-A- 1 690 469
- DE-A1- 3 338 916
- FR-A- 2 800 249
- GB-A- 1 203 938
- US-A- 1 825 929
- US-A- 4 819 291
- US-A- 5 253 386
- US-A- 5 297 310
- US-A- 5 733 288
- US-A1- 2004 158 945

## Description

### TECHNICAL FIELD

The present invention relates to a medical implant cleaning tool or a medical debridement tool.

### BACKGROUND OF THE INVENTION

Many medical implants, such as e.g. dental implants, orthopedic implants and vascular stents, are metallic, i.e. they are made of a metal material. Examples of metal materials commonly utilized for constructing metallic medical implants are steel, titanium, zirconium, tantalum, niobium, hafnium and alloys thereof. In particular, titanium and titanium alloys have proved to be suitable to utilize for constructing medical implants. This is due to the fact that titanium is biocompatible, it has excellent corrosion resistance in body fluids, it resists adherence of bacteria, and it is light and strong.

Dental implants are utilized in dental restoration procedures in patients having lost one or more of their teeth, A dental implant comprises, a dental fixture, which is utilized as an artificial tooth root replacement. Thus, the dental fixture serves as a root for a new tooth. The dental fixture is typically a screw, i.e. it has the shape of a screw, and it is typically made of titanium, a titanium alloy, zirconium or a zirconium alloys. The screw is surgically implanted into the jawbone, where after the bone tissue grows around the screw and the screw is fixated in the bone with the bone in close contact with the implant surface. This process is called osseointegration, because osteoblasts grow on and into the surface of the implanted screw. By means of the osseointegration, a rigid installation of the screw is obtained.

Once the implant screw is firmly anchored in the jawbone, it may be elongated by attachment of an abutment to the screw. The abutment, may, just as the screw, be made of titanium, a titanium alloy, zirconium or a zirconium alloy. The Shape and size of the utilized abutment are adjusted such that it precisely reaches up through the mucosa after attachment to the screw. A dental restoration such as a crown, bridge or denture may then be attached to the abutment.

Alternatively, the implant screw has such a shape and size that it reaches up through the mucosa after implantation, whereby no abutment is needed and a dental restoration such as a crown, bridge or denture may be attached directly to the screw.

Orthopedic implants are utilized for the preservation and restoration of the function in the musculoskeletal system, particularly joints and bones, including alleviation of pain in these structures. Vascular stents are tubular implants arranged for insertion into blood vessels in order to prevent or counteract a localized flow constriction, i.e. they counteract significant decreases in blood vessel diameter.

The surface of medical implants such as e.g. dental implants, orthopedic implants and vascular stents, or the vicinity thereof, has sometimes to be cleaned after placing. This is particularly important when an infection or contamination occurs, causing a progressive degenerative process in the bone adjacent to the implant known as periimplantitis. In these cases the surface of the ailing implant has to be cleaned from microbes and contaminants to stop the progression of the disease and ensure re-integration of the implant. Failure to clean the implant surface will eventually lead to loss of bone and implant, and make further alternative treatments difficult and sometimes even impossible. Furthermore, the surface of vascular stents may have to be cleaned during implantation in order to remove coagulum, and the interior of vascular stents, i.e. the cavity within vascular stents, may have to be cleaned in an endoscopic procedure during a later treatment due to restenosis, i.e. blocking of the blood vessel.

Traditionally, the dentists and surgeons utilize cleaning tools that are relatively hard, i.e. they have a high hardness degree, in order to provide a thorough cleaning of the metallic medical implant during e.g. surgery, implantation or other treatments. Such hard cleaning tools may, for example, be made of stainless steel, hard metal alloys or hard polymers. However, such hard cleaning tools are not suitable to utilize for cleaning all metallic implant materials. For example, they are not suitable to utilize for cleaning medical implants of soft metals or metal alloys, such as e.g. titanium, a titanium alloy, zirconium or a zirconium alloy. This is due to the fact that such medical implants have a delicate surface that may be damaged when contacted by hard cleaning tools. Thus, when hard cleaning tools are utilized for cleaning a medical implant of, for example, titanium, a titanium alloy, zirconium or a zirconium alloy there is a great risk that the surface of the medical implant is damaged by the cleaning process. Then the surface structure of the medical implant is negatively affected. In addition, any produced scratches in the medical implant surface may constitute sites in which bacteria may adhere, which may result in reinfections in the tissue surrounding the medical implant, e.g. the gingiva.

US 5,699,578 discloses a cleaning device for use in the interdental area, comprising two twisted, wire-type sections into which radially extending fibers are inserted.

Furthermore, the above mentioned hard cleaning tools may contaminate a delicate surface of a medical implant when utilized for cleaning the medical implant surface, i.e. they may leave contaminating material residues on the medical implant surface. These material residues often trigger a foreign body response and are generally not well accepted by the human body.

In order to avoid the above mentioned damaging risk, a cleaning tool in the form of a brush comprising soft bristles may be utilized instead of the above mentioned hard cleaning tools for cleaning metallic medical implants having delicate surfaces. The soft bristles may then be made of e.g. a plastic material, nylon or any other synthetic fibres. One example of such a brush for cleaning a dental implant is disclosed in US 6,345,406. However, the cleaning effect of such brushes on the medical implant surface is not as good as that of hard cleaning tools, i.e. it is easier to clean more efficiently and thoroughly by means of hard cleaning tools. In addition, in case such a brush is utilized for cleaning a medical implant surface, it is common that one or more soft bristles, or parts thereof, come loose from the brush and get stuck in the surrounding tissue, e.g. mucosa, whereby inflammation or infections often results.

Thus, there is still a need for a medical implant cleaning tool, by which metallic medical implants of hard metal materials as well as soft metal materials may be cleaned and by which the above mentioned drawbacks are eliminated or at least essentially reduced, i.e. a device that is hard enough to clean well without damaging a delicate medical implant surface and that does not leave contaminants incompatible with reintegration of the implanted structure.

In addition, for different reasons it may be advantageous or necessary to debride surgically exposed hard tissue surfaces. For example, debriding of surgically exposed hard tissue surfaces may be advantageous or necessary to perform before regenerative treatment, i.e. in order to prepare the hard tissue surfaces for regenerative treatment. Example of conditions, which may be associated with a treatment in which debridement of a surgically exposed hard tissue surface is advantageous or necessary to perform in order to prepare the surface for regenerative treatment, are: periimplantitis, periodontitis lesions, marginal periodontitis, apical periodontitis, furcation defects, apical granulomas and cysts, bone cysts, bone tumours, bone granulomas, bone cancers, (infected) extraction sockets, alveolitis sicca ("dry socket"), cleaning of apicectomy defects, localized osteomyelitis, trauma induced defects, resection or revision of implants, resection or revision of fractures, and removal of temporary bone implants (such as orthopaedic bone plates, retainers and screws). Furthermore, debridement of articular surfaces in joints affected by arthritis and debridement of such surfaces before regenerative treatment for cartilage and ligaments is instituted may also be advantageous or necessary to perform.

It is a well-known fact that the morbidity and frequency of adverse effects, such as e.g. post-surgery effects, are directly related to, and often proportional to, the time used for the debridement of surgically exposed hard tissue surfaces. Thus, rapid debridement treatment ensures a better total treatment outcome. In addition, the total treatment outcome may also depend on the degree of damaging of the anatomical structure by the debridement tool during the debridement procedure. Furthermore, the total treatment outcome may also depend on the amount of contaminating material residues that is left on the treated surface by the debridement tool. Contaminating material residues may trigger a foreign body response.

Thus, there is also a need for a medical debridement tool, which is hard enough to debride well without damaging a treated surgically exposed hard tissue surface, which does not leave contaminants on a treated surgically exposed hard tissue surface such that a foreign body response is triggered and which may be utilized for relatively rapid debridement treatment of a surgically exposed hard tissue surface.

### SUMMARY OF THE INVENTION

Accordingly, one object of the present invention is to provide an improved medical implant cleaning tool for cleaning a metallic medical implant.

This object is achieved in accordance with the characterizing portion of claim 1.

Another object of the present invention is to provide an improved medical debridement tool.

This object is achieved in accordance with the characterizing portion of claim 24.

Preferred embodiments are listed in the dependent claims.

Still other objects and features of the present invention will become apparent from the following detailed description considered in conjunction with the accompanying drawings. It is to be understood, however, that the drawings are designed solely for purposes of illustration and not as a definition of the limits of the invention, for which reference should be made to the appended claims. It should be further understood that the drawings are not necessarily drawn to scale and that, unless otherwise indicated, they are merely intended to conceptually illustrate the structures described herein.

### BRIEF DESCRIPTION OF THE DRAWINGS

In the drawings, wherein like reference characters denote similar elements throughout the several views:
Figure 1 shows schematically a first embodiment of a medical implant cleaning tool or medical debridement tool according to the invention;
Figure 2 shows schematically a first embodiment of the medical implant cleaning tool or medical debridement tool according to the invention with a linking component;
Figure 3 shows schematically a second embodiment of the medical implant cleaning tool or medical debridement tool according to the invention,
Figure 4 shows schematically a third embodiment of the medical implant cleaning tool or medical debridement tool according to the invention, and
Figure 5 shows schematically a fourth embodiment of the medical implant cleaning tool or medical debridement tool according to the invention.

### DETAILED DESCRIPTION OF PREFERRED EMBODIMENTS

The present invention provides a medical implant cleaning tool for cleaning a metallic medical implant. In the present context, the term "metallic medical implant" means a medical implant which mainly comprises metal components. Furthermore, in the present context, the term "medical implant" includes within its scope any device intended to be implanted into the body of a vertebrate animal, in particular a mammal such as a human. Medical implants may also be denoted as medical prosthetic devices. Generally, a medical implant is composed of one or several implant parts. Non-limiting examples of medical implants are medical devices that replace anatomy and/or restore a function of the body such as e.g. dental implants, orthopedic implants and vascular stents.

In the present context, the term "dental implant" includes within its scope any device intended to be implanted into the oral cavity of a vertebrate animal, in particular a mammal such as a human, in tooth restoration procedures. Dental implants may also be denoted as dental prosthetic devices. Generally, a dental implant is composed of one or several implant parts. For instance, a dental implant usually comprises a dental fixture coupled to secondary implant parts, such as an abutment and/or a dental restoration such as a crown, bridge or denture. However, any device, such as a dental fixture, intended for implantation may alone be referred to as an implant even if other parts are to be connected thereto.

In the present context, the term "orthopedic implant" includes within its scope any device intended to be implanted into the body of a vertebrate animal, in particular a mammal such as a human, for preservation and restoration of the function of the musculoskeletal system, particularly joints and bones, including the alleviation of pain in these structures. Non-limiting examples of orthopedic implants are hip-joint prostheses, knee prostheses, elbow prostheses, finger prostheses, cochlear prostheses, and fixation screws.

In the present context, the term "vascular stent" refers to a tubular implant arranged for insertion into blood vessels of a vertebrate animal, in particular a mammal such as a human, in order to prevent or counteract a localized flow constriction, i.e. in order to counteract significant decreases in blood vessel diameter.

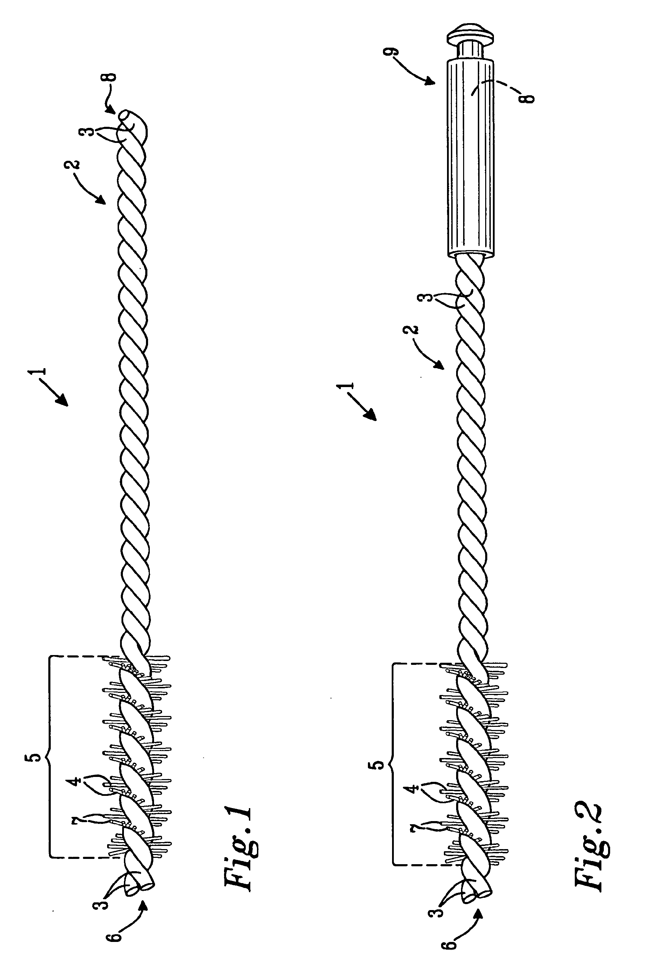

Figure 1 shows schematically a first embodiment of a medical implant cleaning tool 1, i.e. a brush, according to the invention. The brush 1 may be utilized for cleaning a metallic medical implant, such as e.g. a dental implant, an orthopedic implant or a vascular stent. However, it has surprisingly been found that the brush 1 may also be utilized for other medical cleaning procedures than cleaning of medical implants. For example, the brush may be utilized for debridement of surgically exposed hard tissue surfaces. Thereby, the brush 1 may also be denoted as a medical debridement tool. This will be further described below. Alternatively, the brush 1 may be denoted as a medical cleaning tool.

The brush 1 comprises an elongated base member 2, which in the first embodiment is formed of two wires 3 being twisted with each other. However, as will be described below, the elongated base member 2 may alternatively be formed of more than two wires 3 being twisted with each other.

Furthermore, the brush 1 comprises a plurality of bristles 4 fixed between the twisted wires 3. Each bristle 4 extends away from the twisted wires 3, i.e. the length of the respective bristles 4 does not extend in the longitudinal direction of the base member 2. The bristles 4 are positioned in a cleaning section 5, i.e. a brush section, at a first end 6 of the base member 2. The brush section 5 may be positioned in the immediate vicinity of the first end 6 of the base member 2, i.e. with no part of the base member 2 between the first end 6 and the brush section 5. Alternatively, there may be a part of the base member 2 between the first end 6 and the brush section 5 (see figure 1).

According to the invention, the bristles 4 consist of titanium or a titanium alloy. The term "alloy" is herein intended to mean a metallic material containing a base metal and at least one alloying component. The term "base metal" is herein intended to mean the metal being the primary constituent of the alloy and the term "alloying component" is intended to mean a component added to the base metal in order to form the alloy. Thus, the term "titanium alloy" is intended to mean an alloy comprising titanium as base metal and at least one alloying component.

Thus, the bristles 4 may consist of pure, i.e. unalloyed, titanium. For example, the bristles 4 may consist of titanium selected from the group consisting of: titanium of grade 1, titanium of grade 2, titanium of grade 3 and titanium of grade 4 according to ASTM F67. These types of titanium are sometimes also denoted as "commercially pure" titanium.

Alternatively, the bristles 4 may consist of a titanium alloy, whereby the titanium alloy comprises titanium as base metal and at least one alloying component selected from the group consisting of: zirconium, tantalum, hafnium, niobium, aluminium, vanadium, molybdenum, chrome, cobalt, magnesium, iron, gold, silver, copper, mercury, tin and zinc.

For example, the bristles 4 may consist of a titanium alloy, whereby the titanium alloy comprises titanium as base metal, and aluminium and vanadium as alloying components. One preferred example of such a titanium alloy comprises about 94.5% titanium, about 3% aluminium and about 2.5% vanadium.

Another example of a titanium alloy, which the bristles 4 may consist of, is a Titanium-6 Aluminium-4 Vanadium (Ti6Al4V) alloy. For example, the bristles 4 may consist of a Titanium-6 Aluminium-4 Vanadium (Ti6Al4V) alloy selected from the group consisting of a Titanium-6 Aluminium-4 Vanadium (Ti6Al4V) alloy according to ASTM F136 and a Titanium-6 Aluminium-4 Vanadium (Ti6Al4V) alloy according to ASTM F1472.

Alternatively, the bristles 4 may consist of a titanium alloy selected from the group consisting of a Titanium-6 Aluminium-7 Niobium (Ti6AI7Nb) alloy according to ASTM F1295, a Titanium-13 Niobium-13 Zirconium (Ti13Nb13Zr) alloy according to ASTM F1713 and a Titanium-12 Molybdenum-6 Zirconium-2 Iron (Ti12Mo6Zr2Fe) alloy according to ASTM F1813.

The wires 3 may consist of any suitable material, such as e.g. a metal or an alloy. However, the wires 3 consist preferably of titanium or a titanium alloy. Thus, the wires 3 may consist of pure, i.e. unalloyed, titanium. For example, the wires 3 may consist of titanium selected from the group consisting of: titanium of grade 1, titanium of grade 2, titanium of grade 3 and titanium of grade 4 according to ASTM F67.

Alternatively, the wires 3 may consist of a titanium alloy, whereby the titanium alloy comprises titanium as base metal and at least one alloying component from the group consisting of: zirconium, tantalum, hafnium, niobium, aluminium, vanadium, molybdenum, chrome, cobalt, magnesium, iron, gold, silver, copper, mercury, tin and zinc.

For example, the wires 3 may consist of a titanium alloy, whereby the titanium alloy comprises titanium as base metal, and aluminium and vanadium as alloying components. One preferred example of such a titanium alloy comprises about 94.5% titanium, about 3% aluminium and about 2.5% vanadium.

Another example of a titanium alloy, which the wires 3 may consist of, is a Titanium-6 Aluminium-4 Vanadium (Ti6AI4V) alloy. For example, the wires 3 may consist of a Titanium-6 Aluminium-4 Vanadium (Ti6AI4V) alloy selected from the group consisting of a Titanium-6 Aluminium-4 Vanadium (Ti6AI4V) alloy according to ASTM F136 and a Titanium-6 Aluminium-4 Vanadium (Ti6AI4V) alloy according to ASTM F1472.

Alternatively, the wires 3 may consist of a titanium alloy selected from the group consisting of a Titanium-6 Aluminium-7 Niobium (Ti6Al7Nb) alloy according to ASTM F1295, a Titanium-13 Niobium-13 Zirconium (Ti13Nb13Zr) alloy according to ASTM F1713 and a.Titanium-12 Molybdenum-6 Zirconium-2 Iron (Ti12Mo6Zr2Fe) alloy according to ASTM F1813.

In the first embodiment the wires 3 are solid, i.e. their interior is completely filled up with the unalloyed titanium material or the titanium alloy that they consist of. Thus, they are not hollow.

The bristles 4 and the wires 3 may consist of the same material, i.e. both the bristles 4 and the wires 3 may consist of e.g. pure (unalloyed) titanium of a certain grade or any of the above mentioned titanium alloys. However, the bristles 4 and the wires 3 may also consist of different materials. For example, the bristles 4 may, thus, consist of pure titanium of a certain grade, while the wires 3 consist of pure titanium of another grade. As an alternative example, the bristles 4 may consist of pure titanium, while the wires 3 consist of a titanium alloy, or vice versa. As a further example, the bristles 4 may consist of a certain titanium alloy, while the wires 3 consist of another titanium alloy.

In addition, the respective wires 3 may consist of different materials. For example, in the first embodiment one wire 3 may consist of one of the above mentioned unalloyed titanium materials or titanium alloys, while the other wire 3 consist of another of the above mentioned unalloyed titanium materials or titanium alloys. Likewise, one or more of the bristles 4 may consist of one of the above mentioned unalloyed titanium materials or titanium alloys, while the other bristles 4 consist of another of the above mentioned unalloyed titanium materials or titanium alloys.

The base member 2 may have a length of e.g. 5-500 mm or 10-500 mm and the brush section 5 may have a length of e.g. 1.0-100 mm or 2-10 mm. The diameter of the respective wires 3 may be e.g. 0.1-2.0 mm or 0.1-1 mm. The bristles 4 may have a length of e.g. 0.1-50 mm or 0.1-10 mm and a diameter of 0.05-1.0 mm or 0.05-0.5 mm.

In the first embodiment shown in figure 1, all bristles 4 have essentially the same length. The outer ends 7 of the bristles 4 form thereby parts of a surface area of a cylinder, whereby the brush section 5 has a cylindrical shape.

The brush 1 according to the invention may be produced by, for example, any known method in which bristles are introduced between wires, where after the wires are twisted with each other. The bristles 4 may, for example, be fixed between the twisted wires 3 by means of interlocking between the twisted wires 3. Alternatively, the bristles 4 may be fixed by means of electric welding or by means of heating to a high temperature.

Furthermore, the brush 1 according to the invention may be intended to be utilized together with a motor-driven unit, such as e.g. a contra-angle handpiece for dental drilling or endodontic work, or an orthopedic drill. The brush 1 may then optionally comprise a linking component 9, for connection to a motor-driven unit. The linking component 9 is provided at a second end 8 of the base member 2. Figure 2 shows schematically a first embodiment of the brush 1 with a linking component 9.

Alternatively, the brush 1 according to the invention may be intended for manual use. The brush may then optionally comprise a handle (not shown) instead of the linking component 9 at the second end 8 of the base member 2.

The brush 1 according to the invention may be utilized during surgery for cleaning of the surface of a metallic medical implant after infection and/or bone resorption. For example, it may be utilized for cleaning the surface of a metallic dental implant or a metallic orthopedic implant. Thus, it may be utilized for removing e.g. bacterial biofilm, debris, calculus or fibrous tissue from the surface of a dental implant, such as a titanium screw. Alternatively, the brush 1 may be utilized together with a cleaning agent (i.e. an antibacterial agent) in order to remove the bacterial biofilm from the vicinity of the dental fixture during implantation. The brush 1 may also be utilized for cleaning the surface of, or the vicinity of, an abutment.

In addition, the brush 1 may be utilized for removing cement remnants, bacterial biofilm, debris, calculus or fibrous tissue from the surface of an orthopedic implant or for removing plaque from the surface of a vascular stent during. Alternatively, it may be utilized for cleaning the interior of a vascular stent, i.e. the cavity within a vascular stent, in an endoscopic procedure during a later treatment due to restenosis, i.e. blocking of the blood vessel.

It has surprisingly been found that the medical implant cleaning tool, i.e. the brush 1, according to the invention is advantageous to utilize for cleaning metallic medical implants. It is advantageous to utilize for cleaning both "hard" metallic medical implants having relatively hard surfaces, such as e.g. medical implants of steel, and "soft" metallic medical implants having delicate surfaces, such as e.g. medical implants of titanium, a titanium alloy, zirconium or a zirconium alloy. This is due to the fact that the parts of the medical implant cleaning tool according to the invention that contact the implant surface in order to perform the cleaning action, i.e. the bristles 4 which are made of titanium or a titanium alloy, have proved to be hard enough to clean both hard and delicate implant surfaces well. At the same time they do not have such hardness that they damage delicate surfaces, i.e. they do not essentially damage delicate surfaces. Consequently, the risk of negatively affecting the surface structure of the medical implant is reduced when the cleaning tool according to the invention is utilized instead of the above mentioned hard cleaning tools. In addition, when the damaging risk is reduced, the risk of formation of scratches constituting bacteria adherence sites is also reduced. Thus, the risk of re-infection in the tissue surrounding the implant, e.g. the gingival, is reduced too.

In addition, the medical implant cleaning tool, i.e. the brush 1, according to the invention does not leave contaminants, i.e. material residues, incompatible with reintegration of the implanted structure. In case a bristle, or parts thereof, come loose, a foreign body response is usually not triggered since titanium is biocompatible. Thus, the inflammation risk due to a loosened piece from the cleaning tool is minimal. In addition, a loosened titanium piece may heal up with the medical implant.

In particular, the medical implant cleaning tool, i.e. the brush 1, according to the invention is well-suited for cleaning a medical implant of titanium or a titanium alloy that has a delicate surface, which according to the above is easily damaged by hard cleaning tools. In addition, the titanium or titanium alloy of which the bristles is made may be selected such that the hardness degree thereof exactly, or at least essentially, correspond to the hardness degree of a titanium implant surface to be cleaned. For example, in case the implant to be cleaned consists of pure titanium, it is preferred to select pure titanium as the material of the bristles 4. Alternatively, in case the implant to be cleaned consists of a specific titanium alloy, it is preferred to select the same titanium alloy as the material of the bristles 4. In addition, the cleaning tool according to the invention is also particularly suitable for cleaning a medical implant of zirconium or a zirconium alloy since the hardness degree of zirconium and titanium are similar.

Furthermore, the shape of the brush 1 is well-suited for cleaning a surface of a screw, e.g. a dental fixture having threads, made of titanium, a titanium alloy, zirconium or a zirconium alloy.

As mentioned above, the brush 1 according to the invention may also be utilized as a medical debridement tool. More specifically, it has surprisingly been found that the brush 1 may be utilized for other medical cleaning procedures than cleaning or debridement of medical implants. In particular, it is well-suited for debridement procedures, such as e.g. debridement of surgically exposed hard tissue surfaces. For example, debriding of surgically exposed hard tissue surfaces may be advantageous or necessary to perform before regenerative treatment, i.e. in order to prepare the hard tissue surfaces for regenerative treatment. Thus, any of the herein described embodiments or variants of the brush 1 is not only a medical implant cleaning tool, but also a medical debridement tool. Alternatively, any of the herein described embodiments or variants of the brush 1 may be denoted as a medical cleaning tool.

Hard tissues are, for example, bone, cementum, dentin, enamel, cartilage and ligaments. The term "debridement" means cleaning of a hard tissue surface in order to remove, for example, biofilm, concrements, microbes, unwanted tissue, cells and cell residues, scar tissue, and/or necrotic tissue. Debridement may, for example, be performed in order to control local infections, inflammations, foreign body reactions, pathological conditions, degenerative processes (e.g. periodontitis, periimplantitis).

The brush 1 according to the invention may be utilized for debridement of surgically exposed hard tissue surfaces in treatment of many different conditions. Non-limiting examples of conditions, which may be associated with a treatment in which debridement of a surgically exposed hard tissue surface may be performed by means of the brush 1 according to the invention in order to prepare the surface for regenerative treatment, are: periimplantitis, periodontitis lesions, marginal periodontitis, apical periodontitis, furcation defects, apical granulomas and cysts, bone cysts, bone tumours, bone granulomas, bone cancers, (infected) extraction sockets, alveolitis sicca ("dry socket"), cleaning of apicectomy defects, localized osteomyelitis, trauma induced defects, resection or revision of implants, resection or revision of fractures, and removal of temporary bone implants (such as orthopaedic bone plates, retainers and screws). Furthermore, debridement of articular surfaces in joints affected by arthritis and debridement of such surfaces before regenerative treatment for cartilage and ligaments is instituted may also be performed by means of the brush 1 according to the invention.

A procedure involving use of the brush 1 according to the invention may, for example, involve the steps of: surgically exposing a hard tissue surface to be treated; removal of inflamed soft tissue; debriding the surface by means of the brush 1 according to the invention; applying (regenerative) treatment as needed; replacing soft tissue; suturing for good primary closure and wound stability; and allowing the wound to heal.

In particular, it has been observed that the brush 1 according to the invention is an efficient tool for debridement of surgically exposed tooth root surfaces, furcation defects and bony defects before regenerative treatment (i.e. by means of, for example Straumann^{®} Emdogain, bone graft materials, autologous bone, membranes, etc.).The brush 1 is especially effective for removing granulation tissue, and for removing concrements of calcified biofilms (plaques) and subgingival calcus.

The brush 1 according to the invention is well-suited for debridement of a surgically exposed hard tissue surface, since the parts of the brush 1 that contact the hard tissue surface in order to perform the debridement action, i.e. the bristles 4 which are made of titanium or a titanium alloy, have proved to be hard enough to clean hard tissues surfaces well. At the same time, the hardness, stiffness and elasticity of the titanium or titanium alloy, of which the bristles 4 are made, are such that the bristles 4 efficiently debride the surface without imposing any damage to the anatomical structure, thus maintaining the outline of the original anatomy even after substantial instrumentation of the surface.

In addition, the brush 1 does not leave any bio-compromising contaminants on the treated hard tissue surface. Titanium is bio-inert and does not provoke any adverse effects. Any titanium or titanium alloy contamination left on the treated surface does not have any substantial clinical consequence. In case a bristle 4 or part thereof come loose, a foreign body response is usually not triggered since titanium is biocompatible. Thus, the inflammation risk due to a loosened piece from the brush 1 is minimal.

Furthermore, a relatively rapid debridement procedure of surfaces, which are otherwise hard to clean and/or hard to reach by hand instrumentation, may be performed by means of the brush 1 according to the invention. Rapid treatment ensures a better treatment outcome. As mentioned above, it is a well-known fact that the morbidity and frequency of adverse effects, such as e.g. post-surgery effects, are directly related to, and often proportional to, the time used for the debridement of surgically exposed hard tissue surfaces. Thus, rapid debridement treatment ensures a better total treatment outcome.

In addition, the material properties of titanium are such that the tip of the bristles 4 may be manufactured with sharp edges that provide a good "cutting edge" that effectively clean away inflamed/infected soft tissue and calculus from the defect area without harming sound/viable bone or other hard tissues.

The mentioned advantages of the brush 1 imply that when the brush 1 is utilized as a medical debridement tool in a treatment involving debriding of a surgically exposed hard tissue surface, a total treatment outcome is improved.

The use of the brush 1 as a medical debridement tool is especially favourable where the treatment plan for a defect includes placing of a titanium implant or any other device made of titanium, since only titanium and no other metallic ions or polymers that can provoke unwanted (adverse) clinical and/or biological effects can contaminate the treated area, hampering the outcome of planned (future) implant procedures.

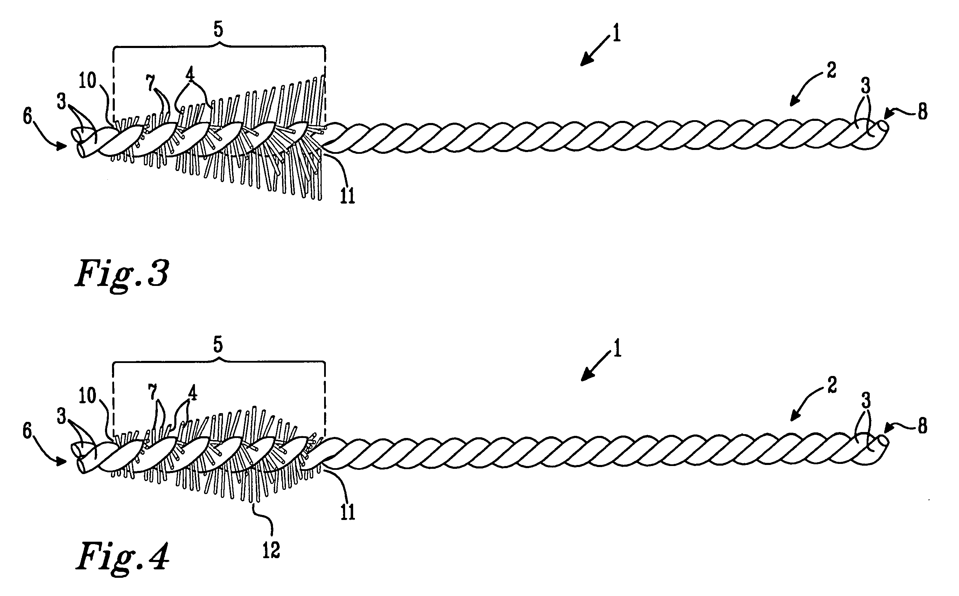

Figure 3 shows schematically a second embodiment of the brush 1 according to the invention. The second embodiment corresponds to the first embodiment except for concerning the length of the bristles 4. In the second embodiment the bristles 4 have a varying length over, i.e. along, the longitudinal direction of the base member 2. More specifically, the bristles 4 have a varying length over the longitudinal direction of the brush section 5. The expression that "the bristles have a varying length over the longitudinal direction" is herein intended to mean that the length of at least some of the bristles 4 is different, i.e. that the length of the bristles 4 differ between at least some positions in the longitudinal direction of the base member 2.

As may be seen in figure 3, the length of the bristles 4 of the second embodiment of the brush 1 increases successively in a direction from a distal end 10 of the brush section 5 (i.e. a brush section distal end 10) to a proximal end 11 of the brush section 5 (i.e. a brush section proximal end 11). The outer ends 7 of the bristles 4 form thereby part of a surface area of a cone, whereby the brush section 5 has a conical shape.

The second embodiment may be varied in accordance with the variations of the first embodiment and has the same advantages as the first embodiment. In addition, it has the advantage that the shape is well suited for deep and wide V-shaped pathological bone pockets. This shape of the second embodiment will ease the cleaning of these V-shaped pockets.

Figure 4 shows a third embodiment of the brush 1 according to the invention. The third embodiment corresponds to the first embodiment except for concerning the length of the bristles 4. In the third embodiment the bristles 4 have a varying length over, i.e. along, the longitudinal direction of the base member 2. More specifically, the bristles 4 have a varying length over the longitudinal direction of the brush section 5.

As may be seen in figure 4, the length of the bristles 4 of the third embodiment of the brush 1 increases successively in a direction from the distal end 10 of the brush section 5 (i.e. the brush section distal end 10) to an intermediate position 12 in the longitudinal direction of the brush section 5. Thereafter the length of the bristles 4 decreases successively in a direction from the intermediate position 12 to the proximal end 11 of the brush section 5 (i.e. the brush section proximal end 11). The outer ends 7 of the bristles 4 form thereby part of a surface area of en element having a diamond-like shape in a side view. Thereby the brush section 5 has a diamond-like shape in a side view.

The third embodiment may be varied in accordance with the variations of the first embodiment and has the same advantages as the first embodiment. In addition, it has the advantage that the shape is well suited for narrow V-shaped pathological bone pockets. The shape of this third embodiment will ease the cleaning of these V-shaped pockets.

In alternative embodiments (not shown) the bristles 4 have a varying length over the longitudinal direction of the base member 2 such that the outer ends 7 of the bristles 4 form part of a surface area of an element having another shape than those mentioned above.

Thus, the size and shape of the brush 1 may be adapted to a defect anatomy, i.e. the size and shape of the brush 1 may be adapted such that it is suited for debriding of a particular type of surgically exposed hard tissue surface. For example, the cleaning section 5 of the brush 1 may be relatively long and narrow (i.e. the bristles 4 may have a relatively short length), whereby the brush 1 is suited for debridement of a surgically exposed hard tissue surface in treatment of, for example, vertical periodontics defects. Alternatively, the cleaning section 5 of the brush 1 may be ball-shaped (not shown). Then the bristles have a varying length over the longitudinal direction of the base member, whereby the length of the bristles increases successively in a direction from a cleaning section distal end to an intermediate position in the cleaning section and decreases successively in a direction from the intermediate position to a cleaning section proximal end such that the cleaning section is ball-shaped. A brush 1 having a ball-shaped cleaning section is suited for debridement of a surgically exposed hard tissue surface in treatment of, for example, granulomas and apical periodontal defects. Furthermore, the cleaning section 5 of the brush 1 may have a conical shape (figure 3), whereby the brush 1 is suited for debridement of a surgically exposed hard tissue surface in treatment of, for example, wide bone defects, such as marginal periodontal defects and dehiscent defects.

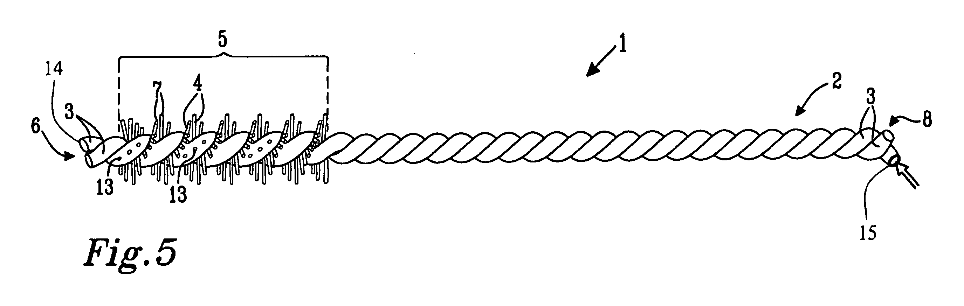

Furthermore, any of the above described embodiments may be varied in that at least one of the wires 3 is a hollow wire comprising apertures in its wall in the cleaning section. Figure 5 shows schematically a fourth embodiment of the brush 1 according to the invention. The fourth embodiment corresponds to the first embodiment except for the fact that one of the two wires 3 is hollow. The hollow wire 3 is open at a wire proximal end 15 and closed at a wire distal end 14, and comprises a plurality of apertures 13 in the wall of the wire 3 within the cleaning section 5. The hollow wire 3 constitutes a pipe for conducting a fluid from the wire proximal end 15 to its apertures 13 for distribution of the fluid from the interior of the hollow wire 3 along the length of at least some of the bristles 4. Thus, the apertures 13 are positioned so as to constitute apertures for distribution of fluid from the interior of the hollow wire 3 along the length of at least some of the bristles 4. The introduction of a fluid into the hollow wire 3 is indicated with an arrow in figure 5. For example, a fluid such as water, sterilized brine, a hydrogen peroxide solution, an antibiotic solution, a weak acid (i.e. maleic acid or formic acid or another weak organic acid) or diluted hydrogen fluoride (0.005-0.1%), may be introduced into the hollow wire 3 during a cleaning operation so as to be distributed along the length of the bristles 4. The fluid may be introduced into the hollow wire 3 in order to irrigate for cooling, for removing debris, for dissolving concrements or mineral precipitations, for flushing the wound and the surface and for killing microbes during cleaning. The fourth embodiment may be further varied in accordance with the variations of the first embodiment. In addition, the fourth embodiment may be varied such that both wires 3 are hollow wires, whereby both wires 3 are open at a wire proximal end 15 and closed at a wire distal end 14 and whereby both wires comprise a plurality of apertures 13 within the brush section 5.

In addition, any of the above described embodiments may be varied such that the elongated base member 2 is formed by more than two wires 3 being twisted with each other. For example, the base member 2 may be formed by three twisted wires 3. The plurality of bristles 4 are then fixed between the plurality of twisted wires 3. In case the brush 1 comprises more than two wires 3, one or more of the wires 3 may be a hollow wire corresponding to the hollow wire described in the fourth embodiment. For example, in case the elongated base member 2 is formed by three wires 3 being twisted with each other, two of the wires 3 may be solid and one wire 3 may be hollow.

Furthermore, the wires 3 in any of the above embodiments need not be completely twisted with each other as in the figures 1-5, i.e. parts of the wires 3 at a section at the second end 8 of the base member 2 (at a proximal section of the cleaning tool 1) may be untwisted (not shown). Untwisted parts of one or more wires 3 may extend away from the remainder wire/wires 3. For example, untwisted parts of a hollow wire 3 may extend away from the other wire(s) so as to allow convenient attachment to e.g. a fluid source. In addition, the wires 3 in any of the above described embodiments need not have the same length, i.e. some or all of them may have different lengths. For example, one of the wires 3 may be shorter than the other(s) (not shown) such that the wire proximal end of the shorter wire is located at another position in the longitudinal direction of the base member 2 than the wire proximal end of the other wire(s). In case the cleaning tool 1 comprises more than two wires, they may all have the same or different lengths. Alternatively, some of them may have the same length. Thus, in variants of the fourth embodiment shown in figure 5, the hollow wire 3 may be shorter or longer than the solid wire 3 and/or extend away from the solid wire 3 so as to allow convenient attachment to e.g. a fluid source.

The invention has been described with reference to the embodied figures. However, the invention is not limited to the above described embodiments. Features from one or more of the above embodiments or variants thereof may be combined as required, and the ultimate scope of the invention should be understood as being defined in the appended claims.

## Claims

1. A medical implant cleaning tool (1) and/or medical debridement tool (1) for cleaning a metallic dental implant in bone, and/or a surgically exposed hard tissue surface,
**characterized in,**
**that** said medical implant cleaning tool (1) and/or medical debridement tool (1) comprises:
an elongated base member (2) formed of at least two wires (3) being twisted with each other, and
a plurality of bristles (4) fixed between said twisted wires (3) and extending away from said twisted wires (3), whereby said bristles (4) are positioned in a cleaning section (5) at a first end (6) of said base member (2); and
**that** said bristles (4) consist of titanium or a titanium alloy,
said medical implant cleaning tool (1) further comprising a linking component (9) for connection to a motor-driven unit, which linking component (9) is provided at a second end (8) of said base member (2).

2. The medical implant cleaning tool (1) and/or medical debridement tool (1) according to claim 1, wherein said motor-driven unit is a contra-angle handpiece for dental drilling or endodontic work, or an orthopedic drill.

3. The medical implant cleaning tool (1) and/or medical debridement tool (1) according to claim 1 or 2,
**characterized in, that**
said bristles (4) consist of a titanium alloy, whereby the titanium alloy comprises titanium as base metal and at least one alloying component selected from the group consisting of: zirconium, tantalum, hafnium, niobium, aluminium, vanadium, molybdenum, chrome, cobalt, magnesium, iron, gold, silver, copper, mercury, tin and zinc.

4. The medical implant cleaning tool (1) and/or medical debridement tool (1) according to claim 2 or 3,
**characterized in, that**
said bristles (4) consist of a titanium alloy, whereby the titanium alloy comprises titanium as base metal and aluminium and vanadium as alloying components.

5. The medical implant cleaning tool (1) and/or medical debridement tool (1) according to any of claims 1-4,
**characterized in, that**
said wires (3) consist of titanium or a titanium alloy.

6. The medical implant cleaning tool (1) and/or medical debridement tool (1) according to claim 5,
**characterized in, that**
said wires (3) consist of a titanium alloy, whereby the titanium alloy comprises titanium as base metal and at least one alloying component selected from the group consisting of: zirconium, tantalum, hafnium, niobium, aluminium, vanadium, molybdenum, chrome, cobalt, magnesium, iron, gold, silver, copper, mercury, tin and zinc.

7. The medical implant cleaning tool (1) and/or medical debridement tool (1) according to claim 6,
**characterized in, that**
said wires (3) consist of a titanium alloy, whereby the titanium alloy comprises titanium as base metal and aluminium and vanadium as alloying components.

8. The medical implant cleaning tool (1) and/or medical debridement tool (1) according to any of the preceding claims,
**characterized in, that**
all of said bristles (4) have essentially the same length, whereby said cleaning section (5) has a cylindrical shape.

9. The medical implant cleaning tool (1) and/or medical debridement tool (1) according to any of claims 1-7,
**characterized in, that**
said bristles (4) have a varying length over the longitudinal direction of said base member (2).

10. The medical implant cleaning tool (1) and/or medical debridement tool (1) according to claim 9,
**characterized in, that**
said bristles (4) have a varying length over the longitudinal direction of said base member (2), whereby the length of said bristles (4) increases successively in a direction from a cleaning section distal end (10) to a cleaning section proximal end (11), whereby said cleaning section (5) has a conical shape.

11. The medical implant cleaning tool (1) and/or medical debridement tool (1) according to claim 9,
**characterized in, that**
said bristles (4) have a varying length over the longitudinal direction of said base member (2), whereby the length of said bristles (4) increases successively in a direction from a cleaning section distal end (10) to an intermediate position (12) in said cleaning section (5) and decreases successively in a direction from the intermediate position (12) to a cleaning section proximal end (11).

12. The medical implant cleaning tool (1) and/or medical debridement tool (1) according to any of the preceding claims,
**characterized in, that**
at least one of said wires (3) is a hollow wire, whereby said hollow wire is open at a wire proximal end (15) and closed at a wire distal end (14), whereby said hollow wire comprises a plurality of apertures (13) within said cleaning section (5), whereby said hollow wire constitutes a pipe for conducting a fluid from said wire proximal end (15) to its respective apertures (13) and whereby said apertures (13) are positioned so as to constitute apertures for distribution of fluid from the interior of said hollow wire along the length of at least some of said bristles (4).

13. The medical implant cleaning tool (1) and/or medical debridement tool (1) according to any of the preceding claims,
**characterized in, that**
at least one of said wires (3) is a solid wire.

14. Use of the medical implant cleaning tool (1) and/or medical debridement tool (1) according to any of claims 1-13 for cleaning and/or debriding a dental implant surface.

15. Use of the medical debridement tool (1) according to claim 14 in treatment of a condition selected from the group of: periimplantitis, infected implants, ailing implants, exposed implants, contaminated implants, or any other conditions where implant structures needs debridement to recover normal function in the body.

16. Use of the medical debridement tool (1) according to any of claims 1-13 for debriding a surgically exposed hard tissue surface.

## Patentansprüche

1. Reinigungswerkzeug (1) für ein medizinisches Implantat und/oder Werkzeug (1) für medizinisches Debridement zum Reinigen eines metallischen Zahnimplantats in Knochen, und/oder in chirurgisch freigelegter Hartgewebeoberfläche, **dadurch gekennzeichnet,**
**dass** das Reinigungswerkzeug (1) für ein medizinisches Implantat und/oder das Werkzeug (1) für medizinisches Debridement umfasst:
ein längliches Basiselement (2), das aus wenigstens zwei Drähten (3), die miteinander verdrillt sind, gebildet ist, und
eine Mehrzahl von Borsten (4), die zwischen den verdrillten Drähten (3) befestigt sind und sich von den verdrillten Drähten (3) weg erstrecken, wobei die Borsten (4) in einem Reinigungsabschnitt (5) an einem ersten Ende (6) des Basiselements (2) positioniert sind; und
**dass** die Borsten (4) aus Titan oder einer Titanlegierung bestehen,
wobei das Reinigungswerkzeug für ein medizinisches Implantat (1) weiterhin eine Verbindungskomponente (9), zur Verbindung zu einem motorgetriebenen Gerät umfasst, wobei die Verbindungskomponente (9) an einem zweiten Ende (8) des Basiselements (2) ausgebildet ist.

2. Reinigungswerkzeug (1) für ein medizinisches Implantat und/oder Werkzeug (1) für medizinisches Debridement nach Anspruch 1,
wobei das motorgetriebene Gerät ein Winkelstück für Dentalbohren oder für Wurzelbehandlung oder ein orthopädischer Bohrer ist.

3. Reinigungswerkzeug (1) für ein medizinisches Implantat und/oder Werkzeug (1) für medizinisches Debridement nach Anspruch 1 oder 2,
**dadurch gekennzeichnet,**
**dass** die Borsten (4) aus einer Titanlegierung bestehen, wobei die Titanlegierung Titan als Basismetall und wenigstens eine Legierungskomponente umfasst, die ausgewählt ist aus der Gruppe bestehend aus: Zirkonium, Tantal, Hafnium, Niobium, Aluminium, Vanadium, Molybdän, Chrom, Kobalt, Magnesium, Eisen, Gold, Silber, Kupfer, Quecksilber, Zinn und Zink.

4. Reinigungswerkzeug (1) für ein medizinisches Implantat und/oder Werkzeug (1) für medizinisches Debridement nach Anspruch 2 oder 3,
**dadurch gekennzeichnet,**
**dass** die Borsten (4) aus einer Titanlegierung bestehen, wobei die Titanlegierung Titan als Basismetall und Aluminium und Vanadium als Legierungskomponenten umfasst.

5. Reinigungswerkzeug (1) für ein medizinisches Implantat und/oder Werkzeug (1) für medizinisches Debridement nach einem der Ansprüche 1-4,
**dadurch gekennzeichnet,**
**dass** die Drähte (3) aus Titan oder einer Titanlegierung bestehen.

6. Reinigungswerkzeug (1) für ein medizinisches Implantat und/oder Werkzeug (1) für medizinisches Debridement nach Anspruch 5,
**dadurch gekennzeichnet,**
**dass** die Drähte (3) aus einer Titanlegierung bestehen, wobei die Titanlegierung Titan als Basismetall und wenigstens eine Legierungskomponente umfasst, die ausgewählt ist aus der Gruppe bestehend aus: Zirkonium, Tantal, Hafnium, Niobium, Aluminium, Vanadium, Molybdän, Chrom, Kobalt, Magnesium, Eisen, Gold, Silber, Kupfer, Quecksilber, Zinn und Zink.

7. Reinigungswerkzeug (1) für ein medizinisches Implantat und/oder Werkzeug (1) für medizinisches Debridement nach Anspruch 6,
**dadurch gekennzeichnet,**
**dass** die Drähte (3) aus einer Titanlegierung bestehen, wobei die Titanlegierung Titan als Basismetall und Aluminium und Vanadium als Legierungskomponenten umfasst.

8. Reinigungswerkzeug (1) für ein medizinisches Implantat und/oder Werkzeug (1) für medizinisches Debridement nach einem der vorhergehenden Ansprüche,
**dadurch gekennzeichnet,**
**dass** alle Borsten (4) im Wesentlichen dieselbe Länge aufweisen, wobei der Reinigungsabschnitt (5) eine Zylinderform aufweist.

9. Reinigungswerkzeug (1) für ein medizinisches Implantat und/oder Werkzeug (1) für medizinisches Debridement nach einem der Ansprüche 1-7,
**dadurch gekennzeichnet,**
**dass** die Borsten (4) über die Längsrichtung des Basiselements (2) eine variierende Länge aufweisen.

10. Reinigungswerkzeug (1) für ein medizinisches Implantat und/oder Werkzeug (1) für medizinisches Debridement nach Anspruch 9,
**dadurch gekennzeichnet,**
**dass** die Borsten (4) über die Längsrichtung des Basiselements (2) eine variierende Länge aufweisen, wobei die Länge der Borsten (4) in einer Richtung von einem distalen Ende (10) des Reinigungsabschnitts zu einem proximalen Ende (11) des Reinigungsabschnitts sukzessiv zunimmt, wobei der Reinigungsabschnitt (5) eine Kegelform aufweist.

11. Reinigungswerkzeug (1) für ein medizinisches Implantat und/oder Werkzeug (1) für medizinisches Debridement nach Anspruch 9,
**dadurch gekennzeichnet,**
**dass** die Borsten (4) über die Längsrichtung des Basiselements (2) eine variierende Länge aufweisen, wobei die Länge der Borsten (4) in einer Richtung von einem distalen Ende (10) des Reinigungsabschnitts zu einer Zwischenposition (12) in dem Reinigungsabschnitt (5) sukzessiv zunimmt und in einer Richtung von der Zwischenposition (12) zu einem proximalen Ende (11) des Reinigungsabschnitts sukzessiv abnimmt.

12. Reinigungswerkzeug (1) für ein medizinisches Implantat und/oder Werkzeug (1) für medizinisches Debridement nach einem der vorhergehenden Ansprüche,
**dadurch gekennzeichnet,**
**dass** wenigstens einer der Drähte (3) ein Hohldraht ist, wobei der Hohldraht an einem proximalen Ende (15) des Drahts offen und an einem distalen Ende (14) des Drahts geschlossen ist, wobei der Hohldraht eine Mehrzahl von Öffnungen (13) in dem Reinigungsabschnitt (5) umfasst, wobei der Hohldraht eine Leitung zum Leiten eines Fluids von dem proximalen Ende (15) des Drahts zu seinen jeweiligen Öffnungen (13) bildet, und wobei die Öffnungen (13) so positioniert sind, dass sie Öffnungen zur Verteilung von Fluid aus dem Inneren des Hohldrahts entlang der Länge wenigstens einiger der Borsten (4) bilden.

13. Reinigungswerkzeug (1) für ein medizinisches Implantat und/oder Werkzeug (1) für medizinisches Debridement nach einem der vorhergehenden Ansprüche,
**dadurch gekennzeichnet,**
**dass** wenigstens einer der Drähte (3) ein Volldraht ist.

14. Verwendung des Reinigungswerkzeugs (1) für ein medizinisches Implantat und/oder des Werkzeugs (1) für medizinisches Debridement nach einem der Ansprüche 1-13 zum Reinigen und/oder Debridieren einer Fläche eines Zahnimplantats.

15. Verwendung des Werkzeugs (1) für medizinisches Debridement nach Anspruch 14 bei einer Behandlung eines Zustands, der ausgewählt ist aus der Gruppe von: Periimplantitis, infizierte Implantate, schmerzende Implantate, freiliegende Implantate, kontaminierte Implantate oder alle anderen Zustände, in denen Implantat-Strukturen ein Debridement benötigen, um eine normale Funktion im Körper wiederzuerlangen.

16. Verwendung des Werkzeugs (1) für medizinisches Debridement nach einem der Ansprüche 1-13 zum Debridieren einer chirurgisch freigelegten Hartgewebeoberfläche.

## Revendications

1. Outil de nettoyage d'implants médicaux (1) et/ou outil de débridement médical (1) pour le nettoyage d'un implant dentaire métallique dans le tissu osseux, et/ou une surface de tissu dur exposée chirurgicalement, **caractérisé en ce que**
ledit outil de nettoyage d'implants médicaux (1) et/ou outil de débridement médical (1) comprend:
un élément allongé de base (2) formé d'au moins deux fils (3) torsadés mutuellement, et
une pluralité de soies (4) fixées entre lesdits fils torsadés (3) et s'étendant à distance desdits fils torsadés (3), lesdites soies (4) étant positionnées dans une section de nettoyage (5) à une première extrémité (6) dudit élément de base (2) ; et **en ce que**
lesdites soies (4) consistent en titane ou en un alliage de titane,
ledit outil de nettoyage d'implants médicaux (1) comprenant en outre un composant de liaison (9) pour la connexion à une unité commandée par moteur, composant de liaison (9) qui est fourni à une seconde extrémité (8) dudit élément de base (2).

2. Outil de nettoyage d'implants médicaux (1) et/ou outil de débridement médical (1) suivant la revendication 1, dans lequel ladite unité commandée par moteur est une pièce à main à contre-angle pour forage dentaire ou travail endodontique, ou bien un foret orthopédique.

3. Outil de nettoyage d'implants médicaux (1) et/ou outil de débridement médical (1) suivant la revendication 1 ou 2,
**caractérisé en ce que**
lesdites soies (4) consistent en un alliage de titane, ledit alliage de titane comprenant ainsi du titane comme métal de base et au moins un constituant d'alliage choisi dans le groupe consistant en : le zirconium, le tantale, l'hafnium, le niobium, l'aluminium, le vanadium, le molybdène, le chrome, le cobalt, le magnésium, le fer, l'or, l'argent, le cuivre, le mercure, l'étain et le zinc.

4. Outil de nettoyage d'implants médicaux (1) et/ou outil de débridement médical (1) suivant la revendication 2 ou 3,
**caractérisé en ce que**
lesdites soies (4) consistent en un alliage de titane, ledit alliage de titane comprenant ainsi du titane comme métal de base, et de l'aluminium et du vanadium comme constituants d'alliage.

5. Outil de nettoyage d'implants médicaux (1) et/ou outil de débridement médical (1) suivant l'une quelconque des revendications 1 à 4,
**caractérisé en ce que**
lesdits fils (3) consistent en titane ou en un alliage de titane.

6. Outil de nettoyage d'implants médicaux (1) et/ou outil de débridement médical (1) suivant la revendication 5,
**caractérisé en ce que**
lesdits fils (3) consistent en un alliage de titane, ledit alliage de titane comprenant ainsi du titane comme métal de base et au moins un constituant d'alliage choisi dans le groupe consistant en : le zirconium, le tantale, l'hafnium, le niobium, l'aluminium, le vanadium, le molybdène, le chrome, le cobalt, le magnésium, le fer, l'or, l'argent, le cuivre, le mercure, l'étain et le zinc.

7. Outil de nettoyage d'implants médicaux (1) et/ou outil de débridement médical (1) suivant la revendication 6,
**caractérisé en ce que**
lesdits fils (3) consistent en un alliage de titane, ledit alliage de titane comprenant ainsi du titane comme métal de base, et de l'aluminium et du vanadium comme constituants d'alliage.

8. Outil de nettoyage d'implants médicaux (1) et/ou outil de débridement médical (1) suivant l'une quelconque des revendications précédentes,
**caractérisé en ce que**
toutes lesdites soies (4) ont pratiquement la même longueur, ladite section de nettoyage (5) ayant ainsi une forme cylindrique.

9. Outil de nettoyage d'implants médicaux (1) et/ou outil de débridement médical (1) suivant l'une quelconque des revendications 1 à 7,
**caractérisé en ce que**
lesdites soies (4) ont une longueur variable dans la direction longitudinale dudit élément de base (2).

10. Outil de nettoyage d'implants médicaux (1) et/ou outil de débridement médical (1) suivant la revendication 9,
**caractérisé en ce que**
lesdites soies (4) ont une longueur variable dans la direction longitudinale dudit élément de base (2), la longueur desdites soies (4) augmentant ainsi de manière successive dans le sens d'une extrémité distale de section de nettoyage (10) à une extrémité proximale de section de nettoyage (11), ladite section de nettoyage (5) ayant ainsi une forme conique.

11. Outil de nettoyage d'implants médicaux (1) et/ou outil de débridement médical (1) suivant la revendication 9,
**caractérisé en ce que**
lesdites soies (4) ont une longueur variable dans la direction longitudinale dudit élément de base (2), la longueur desdites soies (4) augmentant ainsi de manière successive dans le sens d'une extrémité distale de section de nettoyage (10) à une position intermédiaire (12) dans ladite section de nettoyage (5) et diminuant de manière successive dans le sens de la position intermédiaire (12) à une extrémité proximale de section de nettoyage (11).

12. Outil de nettoyage d'implants médicaux (1) et/ou outil de débridement médical (1) suivant l'une quelconque des revendications précédentes,
**caractérisé en ce que**
au moins un desdits fils (3) est un fil creux, ledit fil creux étant ainsi ouvert à une extrémité proximale de fil (15) et fermé à une extrémité distale de fil (14), ledit fil creux comprenant ainsi une pluralité d'orifices (13) dans ladite section de nettoyage (5), ledit fil creux constituant ainsi un conduit pour amener un fluide de ladite extrémité proximale de fil (15) à ses orifices respectifs (13), et lesdits orifices (13) étant ainsi positionnés de manière à constituer des orifices pour la distribution de fluide à partir de l'intérieur dudit fil creux le long d'au moins certaines desdites soies (4).

13. Outil de nettoyage d'implants médicaux (1) et/ou outil de débridement médical (1) suivant l'une quelconque des revendications précédentes,
**caractérisé en ce que**
au moins un desdits fils (3) est un fil plein.

14. Utilisation de l'outil de nettoyage d'implants médicaux (1) et/ou de l'outil de débridement médical (1) suivant l'une quelconque des revendications 1 à 13 pour le nettoyage et/ou le débridement d'une surface d'implant dentaire.

15. Utilisation de l'outil de débridement médical (1) suivant la revendication 14 dans le traitement d'une affection choisie dans le groupe consistant en : une périimplantite, des implants infectés, des implants douloureux, des implants exposés, des implants contaminés, ou n'importe quelle autre affection dans lesquelles des structures d'implants nécessitent un débridement pour rétablir une fonction normale dans l'organisme.

16. Utilisation de l'outil de débridement médical (1) suivant l'une quelconque des revendications 1 à 13 pour le débridement d'une surface de tissu dur exposée chirurgicalement.
